# EUROPÄISCHE PATENTSCHRIFT

(11) **EP 1 357 878 B1**
(45) Veröffentlichungstag und Bekanntmachung des Hinweises auf die Patenterteilung: **02.08.2006**
(21) Anmeldenummer: 02722044.1
(22) Anmeldetag: 05.02.2002
(51) Int. Cl.: A61F 17/00, B65D 43/16

(54) **KRAFTFAHRZEUG-VERBANDMATERIALBEHÄLTER**
MOTOR-VEHICLE CONTAINER FOR DRESSING MATERIAL
CONTENANT DE MATERIEL DE PANSEMENT DE VEHICULE AUTOMOBILE

(30) Priorität: 05.02.2001 DE 10105115
(43) Veröffentlichungstag der Anmeldung: 05.11.2003
(73) Patentinhaber: Paul Hartmann Aktiengesellschaft, 89522 Heidenheim (DE)
(72) Erfinder: KOBSIK, Ralph, 80638 München (DE); MERKLE, Regina, 73450 Neresheim (DE)
(74) Vertreter: Langöhrig, Angelika Beate
(86) Internationale Anmeldenummer: PCT/EP2002/001152
(87) Internationale Veröffentlichungsnummer: WO 2002/062280

(56) Entgegenhaltungen:
- EP-A- 0 520 916
- EP-A- 0 691 114
- WO-A-96/04176
- CH-A- 326 400
- US-A- 2 687 157
- US-A- 3 327 841
- US-A- 3 504 787
- US-A- 3 795 265
- US-A- 5 411 140
- US-A- 5 515 974

## Beschreibung

Die Erfindung betrifft einen Kraftfahrzeug-Verbandmaterialbehälter mit Erste-Hilfe-Material befüllbar, umfassend ein Behälterunterteil zur Aufnahme von Erste-Hilfe-Material mit einem Behälterboden und daran anschließenden Seitenwänden zur Bildung des Behälters sowie einem Behälterdeckel, der an dem Behälterunterteil angelenkt und zwischen einer geschlossenen Position, in der er auf dem Behälterunterteil aufliegt und dieses verschließt, und einer geöffneten Position, in der das Behälterunterteil zugänglich und unverschlossen ist, verschwenkbar ist und wobei der Behälterdeckel in der geschlossenen Position lösbar verriegelbar ist.

Derartige Kraftfahrzeug-Verbandmaterialbehältnisse sind im Stand der Technik vielfach bekannt und werden beispielsweise in der DIN 13 164 definiert hinsichtlich ihrer Merkmale.

Derartige Autoverbandskästen weisen zumeist einen separaten Deckel auf, der über ein Einrastscharnier mit dem Behälterunterteil verbunden ist. Der Deckel ist hierbei flach ausgebildet und verschließt das Behälterunterteil durch Auflegen auf dessen Seitenwände.

Beispielsweise ist im Rahmen der DIN-Norm festgelegt, daß der Behältnisdeckel bis zur Auflagefläche des Behältnisunterteils klappbar sein muß und aus welchen Materialien der Verbandskasten bestehen kann, um beispielsweise eine Beständigkeit gegen Kraftstoffe zu gewährleisten.

Nachteilig bei den bisher bekannten Verbandskästen ist jedoch, daß durch das tiefen Behältnisunterteil die Verbandsmaterialien mehrschichtig übereinander angeordnet werden müssen, so daß kein schneller Zugriff auf das benötigte Material möglich ist und nicht benötigtes, eventuell schon teilweise ausgepacktes Material zur Seite gelegt werden muß und so möglicherweise verschmutzt. Darüber hinaus ist auch die Übersichtlichkeit in einem derartigen Verbandsmaterialbehältnis stark eingeschränkt.

Durch die hohe Kastenform ist darüber hinaus eine Ineinanderstapelfähigkeit im geöffneten Leerzustand nicht möglich oder zumindest nicht eine Stapelbarkeit auf platzsparende Weise, so daß für den Transport leerer Verbandsmaterialkästen eine relativ große Palettenfläche benötigt wird.

Es ist daher Aufgabe der Erfindung, einen Verbandsmaterialbehälter bereitzustellen, der im geöffneten Leerzustand platzsparend stapelbar ist, das darin verstaute Verbandmaterial übersichtlich darbietet und im geöffneten Zustand zugänglich macht und gleichzeitig ein robustes und formschönes Äußeres besitzt.

Die Erfindung löst diese Aufgabe durch einen Kraftfahrzeug-Verbandmaterialbehälter, bei dem der Behälterdeckel als Behälteroberteil zur Aufnahme von Erste-Hilfe-Material ausgebildet ist und im Behälteroberteil befindliches Erste-Hilfe-Material beim Öffnen des Behälters zusammen mit und in dem Behälteroberteil verschwenkbar ist.

Dadurch, daß der Behälterdeckel als Behälteroberteil und damit ebenfalls als Aufnahme für Verbandmaterial ausgebildet ist, kann das aufzunehmende Verbandmaterial besser in den beiden Behälterteilen verstaut werden. Behälteroberteil und Behälterunterteil können dabei so gestaltet sein, daß sie in Richtung auf den Behälterboden bzw. auf den Behälterdeckel so gestaltet sind, daß die Seitenwände zumindest leicht konisch zulaufen, so daß eine Stapelbarkeit der jeweiligen Teile ineinander ermöglicht wird. Sofern es sich um Spritzgußteile handelt, wird dadurch darüber hinaus die Entformbarkeit verbessert.

Insbesondere betrifft die Erfindung befüllte Kraftfahrzeug-Verbandmaterialbehälter.

Es kann insbesondere vorgesehen sein, daß das Behälteroberteil bzw. Behälterunterteil einen Behälterdeckel bzw. einen Behälterboden sowie Seitenwände umfaßt und daß die korrespondierenden Seitenwände des Behälterunterteils und des Behälteroberteils im Bereich ihrer freien Kanten im geschlossenen Zustand des Behälters dichtend gegeinander anliegen.

Diese Abdichtung im geschlossenen Zustand ist notwendig, um den Zutritt von Flüssigkeit beispielsweise im Falle von Regen, aber auch im Falle von austretendem Kraftstoff, zu in dem Behältnis gelagerten Verbandsmaterial sicher zu verhindern.

Es kann dazu vorgesehen sein, daß die Seitenwände des Bodens bzw. die Seitenwände des Deckels mit einer Dichtung versehen sind. Alternativ kann jedoch auch vorgesehen sein, daß sowohl die Wände des Oberteils als auch die Wände des Unterteils im Bereich der freien Kante, mit dem sie gegeneinander anliegen, eine Stufe umfassen, so daß sich die freien Kanten der Seitenwände gegen die jeweilige Stufe der anderen korrespondierenden Seitenwand abstützen und ein Zwischenbereich gegeben ist, in dem sich die Seitenwand des Oberteils und die Seitenwand des Unterteils überlappen. Es wird dadurch eine Art Labyrinthdichtung verwirklicht.

Es kann dabei vorgesehen sein, daß das Behälteröberteil mit dem Behälterunterteil über eine formschlüssige Verbindung z. B. eine Clipsverbindung lösbar verriegelbar ist. Denkbar ist jedoch auch eine materialschlüssige oder andere kraft- oder formschlüssige Verbindung, beispielsweise mittels einer Klettverbindung, wobei ein Velcro-Oberteil an einem Verbindungselement, beispielsweise einer Lasche, beispielsweise am Behälteroberteil angebracht ist und mit einem Velcro-Unterteil zusammenwirkt, das beispielsweise an der Außenseite einer Seitenwand im Bereich des Behälterunterteils befestigt ist.

Es kann im übrigen vorgesehen sein, daß ein Behälterteil mindestens eine Lasche aufweist, die eine Rastnase besitzt, die mit einer korrespondierenden Kante des anderen Behälterteils schnappend, rastend oder in sonstiger Weise hintergreifend zusammenwirkt und auf diese Weise eine formschlüssige Verbindung der beiden Teile sichert, so daß die beiden Teile miteinander gegen unbeabsichtigtes Öffnen verriegelt werden können, wobei ein Öffnen des Behälters durch Entriegeln der formschlüssigen Verbindung möglich ist. Die Entriegelung kann z. B. durch elastische Verformung z. B. der Lasche erfolgen.

Zur verbesserten Stapelbarkeit kann insbesondere vorgesehen sein, daß das Behälterunterteil und das Behälteroberteil im wesentlichen identisch geformt sind und insbesondere im wesentlichen die gleichen Volumina umfassen. Die Teile können dann im aufgeklappten Zustand durch den im wesentlichen symmetrischen Aufbau, sowohl wenn sie miteinander verbunden sind, als auch einzeln, besonders platzgünstig gestapelt werden, so dass ein logistischer Vorteil besteht. Der Palettenbedarf beim Transport der leeren Verbandmaterialbehälter kann dadurch deutlich reduziert werden. Dadurch daß die Behälterhälften bei gleichem Aufnahmevolumen nur halb so hoch ausgebildet zu sein brauchen, verbleibt bei nur geringfügiger Konizität ein geringerer Leerraum, d. h. die Teile können dichter ineinandergeschoben werden. Die störenden flachen Deckel entfallen.

Es kann insbesondere vorgesehen sein, daß das Behälterunterteil und das Behälteroberteil einstückig miteinander verbunden sind. Die beiden Teile können beispielsweise mittels Tiefziehverfahren oder Spritzgußverfahren hergestellt werden und über ein Filmscharnier miteinander verbunden sein. Es kann jedoch nicht nur die gelenkige Verbindung zwischen den beiden Behälterteilen über ein Filmscharnier realisiert werden, darüber hinaus kann auch die Lasche zur Verriegelung von Behälteroberteil und Behälterunterteil miteinander mit dem entsprechenden Behälterteil einstückig über ein Filmscharnier verbunden sein. Hierdurch kann der Montageaufwand zur Herstellung der Kraftfahrzeug-Verbandmaterialbehälter weiter reduziert werden.

Derartige Teile sind verhältnismäßig einfach und kostengünstig herzustellen.

Insbesondere kann der Behälter so gestaltet sein, daß die beiden Behälterteile als Halbschalen gebildet sind, d. h. daß der Übergang zwischen Boden und Deckel und Seitenwänden verrundet ausgebildet ist und weniger eine kastenartige Form als die Form von zwei Schalen besitzt, die aufeinander aufgesetzt werden. Hierdurch wird neben einer entsprechenden Formgebung auch die Stapelbarkeit weiter verbessert.

Insbesondere kann das in den Behälterteilen angeordnete Erste-Hilfe-Material unterverpackt und/oder sortiert in getrennten Inhaltsverpackungen angeordnet sein. Z. B. kann in sterile und unsterile Erste-Hilfe-Materialien unterschieden werden. Insbesondere kann das gesamte Material in einer Behälterseite als Ganzes unterverpackt sein. Der Inhalt ist dadurch übersichtlich angeordnet, wodurch ein verbesserter und sicherer Umgang mit dem z.T. sterilen Verbandsmaterial erzielt wird. Außerdem lassen sich die Unterverpackungen leicht als Set austauschen.

Es kann des weiteren in dem jeweiligen Behälterteil herausnehmbar festgelegt sein. Durch diese Fixierung der Inhaltverpackungen in dem Behältnis verbleiben die jeweiligen Verpackungen in ihrer Behältnishälfte auch beim Öffnen des Verbandmaterialsbehälters. D. h., wird der Deckel entlang seiner gelenkigen Verbindung verschwenkt, so daß er ebenfalls wie das Behälterunterteil auf einer Fläche aufliegt, so verbleibt dennoch das Verbandmaterial, das im Behälteroberteil angeordnet ist, während des gesamten Verschwenkprozesses im Behälteroberteil und fällt nicht heraus oder liegt auf dem Behälterunterteil, von dem es dann erst wieder in das Behälteroberteil umsortiert werden muß. Dies bietet weiterhin den Vorteil, daß die einander gegenüberliegenden Seiten der Inhaltsverpackungen, die im geschlossenen Zustand aufeinander aufliegen, für einen schnelleren Zugriff auf das Verbandmaterial mit Abbildungen der Inhaltsteile, Produktbezeichnungen und gegebenenfalls Anwendungstips bedruckt sein können. Die Fixierung ist dabei so gestaltet, daß ein Austausch der Inhaltsverpackungen teilweise oder vollständig möglich ist. Die Inhaltsverpackungen können dabei kraft-, form- oder materialschlüssig mit dem jeweiligen Behälterteil verbunden sein. Als Verbindungsmöglichkeiten kommen beispielsweise Druckknöpfe, Haftkleber, Klettverbindungen oder Kreuzschlitz/Dornkombinationen in Frage, wobei jeweils ein Teil der Verbindungselemente, außer beim Haftkleber, an der Innenverpackung angeordnet ist und der andere Teil sich im jeweiligen Behälterteil befindet. Die Inhaltsverpackungen können schließlich auch wiederverschließbar sein.

Es kann schließlich vorgesehen sein, daß der Verbandmaterialbehälter aus einem Polypropylenmaterial besteht. Ein derartiger Verbandmaterialbehälter erfüllt die deutschen Normen hinsichtlich eines Verbandskastens und kann mit den darin festgelegten Verbandmaterialien gefüllt sein.

Die Halbschalen können dabei im modernen Design gestaltet sein, insbesondere kann das Material, aus dem das Behältnis besteht, auch teilweise durchscheinend und/oder eingefärbt sein und so eine ansprechende Optik besitzen.

Schließlich kann die Verschlußklappe/Verschlußlasche derart ausgeformt sein, daß diese als Standhilfe dient. So kann der Verbandmaterialbehälter auf die Lasche aufgestellt werden, so daß der Behälter auf einer Schmalseite zu stehen kommt und so z. B. die Sichtseite mit Aufdruck displayartig präsentiert werden kann.

Weitere Merkmale der Erfindung ergeben sich aus den übrigen Anmeldungsunterlagen. Im Folgenden soll anhand einer Zeichnung die Erfindung näher erläutert werden. Dabei zeigen:
- Figur 1: Einen erfindungsgemäßen Kraftfahrzeug-Verbandmaterialbehälter im aufgeklappten Zustand in einer Draufsicht,
- Figur 2: den Kraftfahrzeug-Verbandmaterialbehälter gemäß Figur 1 geschnitten,
- Figur 3 a-c: den Kraftfahrzeug-Verbandmaterialbehälter gemäß Figur 1 im geschlossenen Zustand in verschiedenen Ansichten,
- Figur 4: eine Stapelanordnung der Kraftfahrzeug-Verbandmaterialbehälter und
- Figur 5: eine schematische Darstellung der Befestigung der Inhaltsverpackung im Behälter.

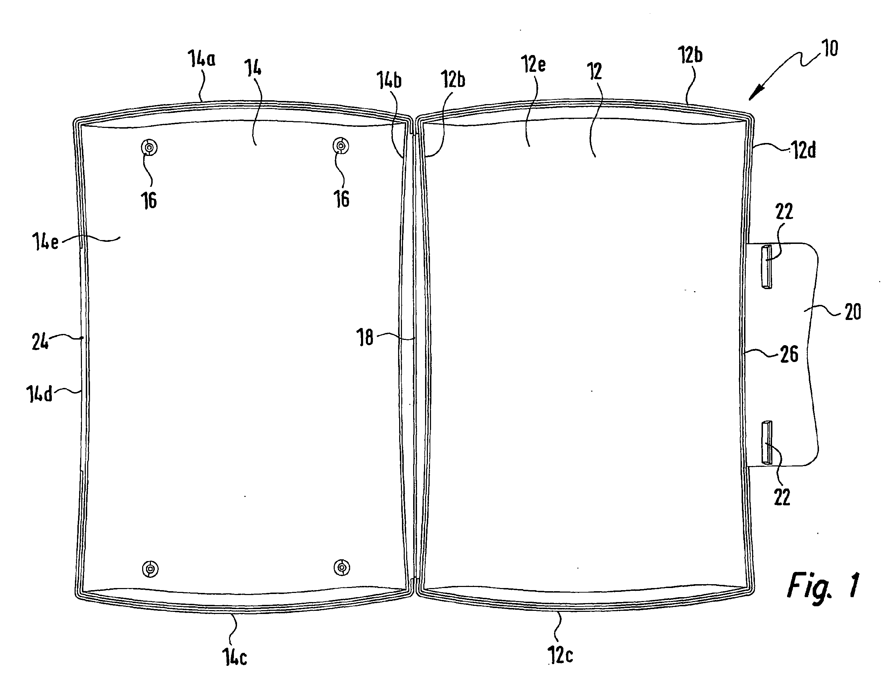

Figur 1 zeigt ein erfindungsgemäßes Kraftfahrzeug-Verbandmaterialbehältnis, das in seiner Gesamtheit mit dem Bezugszeichen 10 versehen ist. Das Kraftfahrzeug-Verbandmaterialbehältnis 10 besteht aus einem Polypropylenmaterial und ist im Spritzgußverfahren hergestellt. Das Polypropylenmaterial ist dabei eingefärbt, wobei es jedoch durchscheinend ist. Der Verbandmaterialbehälter besteht aus einem Behälteroberteil 12 und einem Behälterunterteil 14, wobei das Behälteroberteil 12 aus einem Deckel 12e sowie vier Seitenwänden 12a, 12b, 12c und 12d besteht. Das Behälterunterteil 14 besteht analog aus einem Behälterboden 14e sowie vier Seitenwänden 14a-d. Es korrespondieren hierbei im geschlossenen Zustand des Behälters 10 jeweils die Seitenwänden mit den gleichen Buchstaben. Der Übergang zwischen dem Behälterboden 14e bzw. dem Behälterdeckel 12e und den Seitenwänden 14d und 14b bzw. 12d und b ist dabei stetig, d. h. ohne Abkantung ausgebildet. Hierdurch ergibt sich ein schalenartiges Aussehen der beiden Behälterteile 12 und 14. Die Behälterseiten 14a und 14c sowie 12a und 12c stehen im wesentlichen im 90°-Winkel zum Behälterdeckel 12e bzw. dem Behälterboden 14e. Der Behälterdeckel 12e sowie der Behälterboden 14e sind dabei nicht gekrümmt, sondern flach.

Die beiden Halbschalen 12 und 14 besitzen dabei im wesentlichen die gleichen Abmessungen, so daß im Verschlußfall die Seitenwände 12a-d mit den Seitenwänden 14a-d aufeinander zu liegen kommen und den Behälter dichtend verschließen. Die Seitenwände überlappen sich dabei in einem Teilbereich und liegen mit ihrer freien Kante gegen eine Stufe in der korrespondierenden Seitenwand an. Auf diese Weise erfolgt eine Abdichtung des Behälters 10.

Durch die Verwendung zweier annähernd identischer Halbschalen kann das in die Halbschalen eingebrachte Erste-Hilfe-Material besondes gut und gleichmäßig verteilt werden. So kann in jeder der Halbschalen Erste-Hilfe-Material vorgesehen sein, das sich beim Öffnen der Halbschalen mit entfaltet, so daß jede der Halbschalen von ihrer offenen Seite her zugänglich ist. Das Verbandmaterial wird auf diese Weise besonders übersichtlich und gut zugänglich dargeboten.

Darüber hinaus können auf beiden Verbandmaterialpackungen mit der Sichtseite in Richtung des Benutzers bei aufgeklapptem Verbandskasten 10 Hinweise über den Inhalt sowie die Verwendungsweise des eingefügten Verbandsmaterials vorgesehen sein.

Das Behälterunterteil weist dabei zum Aufstellen auf eine Unterlage vier Füße 16 auf, die auf der Außenseite des Behälterbodens 16e angeordnet sind, wobei die Füße 16 einstückig mit dem Behälterboden verbunden sind. Mittels dieser vier Füße 16 wird eine besonders gute Standfestigkeit des Behälters 10 erzielt.

Die Behälterteile 12 und 14 sind im Bereich ihrer Seitenwände 12b bzw. 14b miteinander gelenkig über ein Filmscharnier 18 verbunden. Das Filmscharnier 18 erstreckt sich dabei über die gesamte Länge der Seitenwände 12b, 14b.

An den Seitenwänden 12d bzw. 14d, die dem Scharnier gegenüberliegen, sind Mittel zum Verriegeln und im geschlossenen Zustand Sichern der beiden Behälterteile 12, 14 angeordnet.

Im Bereich der Seitenwand 12d ist eine Verschlußlasche 20 angebracht, die zwei Rastnasen 22 aufweist, die parallel zur Seitenwand 12d verlaufen und einen L-förmigen Querschnitt aufweisen, wobei sich der lange Schenkel parallel zur Lasche 20 erstreckt und der kurze Schenkel mit seiner freien Seite mit der Lasche 20 einstückig verbunden ist.

Im Gegenzug weist die Seite 14d des Behälterunterteils einen hierin eingeformten Vorsprung 24 auf, der durch die Rastnasen 22 beim Verschließen hintergriffen wird.

Die Lasche 20 ist dabei ebenfalls über ein Filmscharnier 26 mit der Seitenwand 12d verbunden, wobei das Filmscharnier 26 derart ausgebildet ist, daß es im geschlossenen Zustand des Behälters, bei dem die Rastnasen 22 den Vorsprung 24 hintergreifen, eine gewisse Vorspannung besitzt, so daß ein selbständiges Herausspringen der Rastnasen 22 aus der Rastposition hinter dem Vorsprung 24 vermieden wird. Zum Öffnen des Behälters muß vom Benutzer diese Vorspannkraft überwunden werden, so daß die formschlüssige Verriegelungseinrichtung des Verbandbehälters 10 geöffnet werden kann. Der Verbandbehälter 10 kann dann durch Verschwenken der beiden Behälterbestandteile 12 und 14 um das Filmscharnier 18 in seine geöffnete Position gebracht werden.

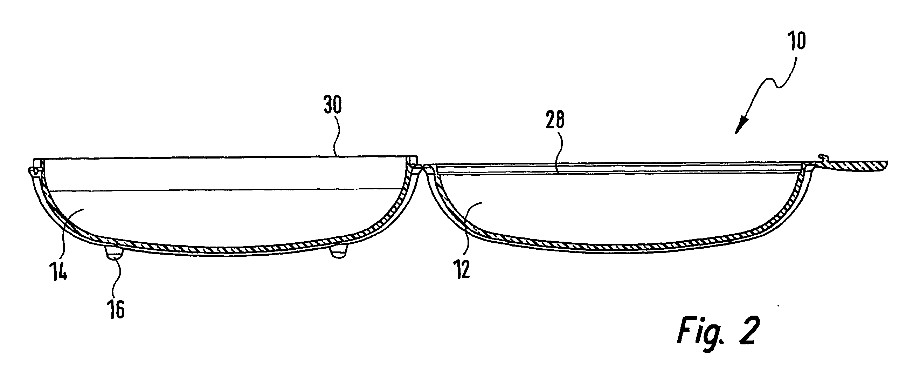

Figur 2 zeigt den Behälter 10 gemäß Figur 1 in einer Schnittdarstellung, wobei hier gut die nach außen ragenden Füße 16 am Behälterunterteil 14 zu erkennen sind, die dem Behälterunterteil eine gute Standfestigkeit sowohl im geöffneten als auch im geschlossenen Zustand verleihen. Ebenfalls gut erkannt werden kann im Bereich des Behälteroberteils 12 eine Stufe 28, gegen die die Oberkante 30 des Behälterunterteils 14 im geschlossenen Zustand anliegt, und über die eine Abdichtung des Behälterinneren gegen Flüssigkeit, wie beispielsweise Regen oder Kraftstoff gewährleistet wird.

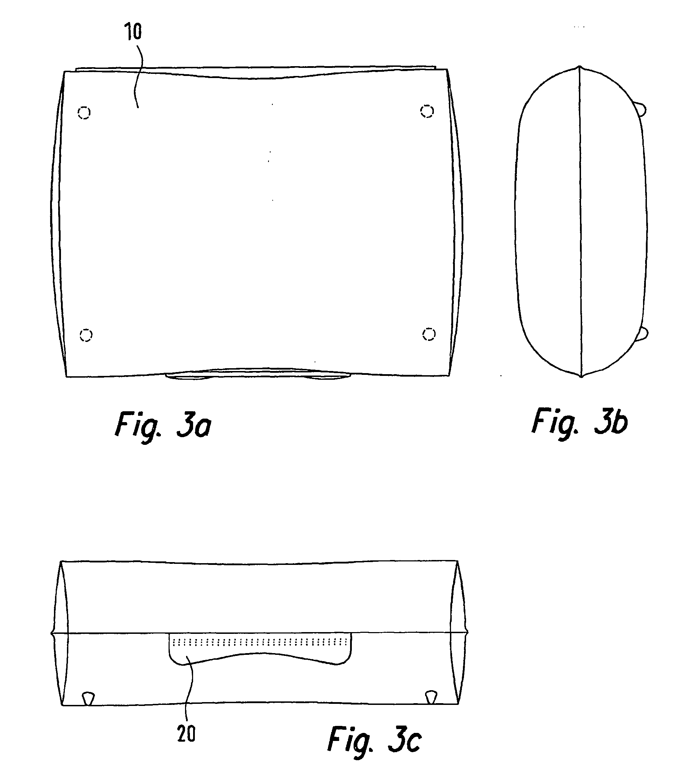

Die Figuren 3a bis 3c zeigen den Behälter in einer Draufsicht, einer Seitenansicht sowie einer Vorderansicht bezüglich seiner Formgebung und seinen Abmaßen im geschlossenen Zustand. Aufgrund seiner geschwungenen Form, die in der Figur 3 erkannt werden kann, besitzt der Behälter 10 ein ansprechendes optisch schönes Gehäuse. Darüber hinaus weist der Behälter abgerundete Kanten auf, die neben einer schöneren Optik auch die Gefahr senken, sich am Verbandskasten zu verletzen.

Die Verriegelungslasche 20 ist am Behälteroberteil 12 angeordnet und verläuft in der Verriegelungsposition, wie sie in Figur 3c dargestellt ist, im wesentlichen so, daß sich die Lasche an die Seitenwand 14d, an der der Vorsprung 24 angeordnet ist, anschmiegt.

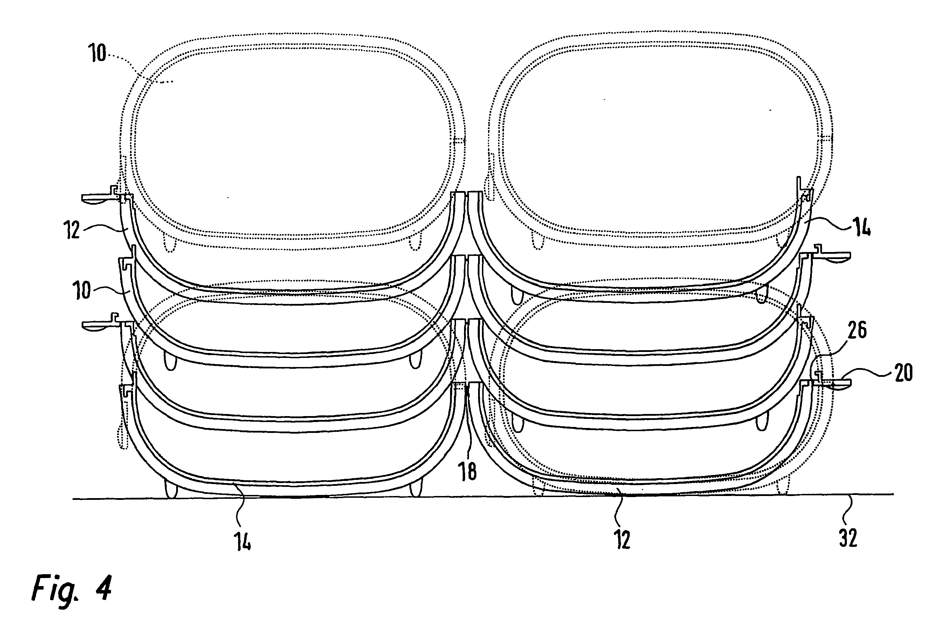

Figur 4 zeigt die erfindungsgemäßen Verbandmaterialbehälter 10 im ineinandergestapelten Zustand, wie sie für eine Lieferung vorbereitet sein können. Es sind hierbei vier ineinandergestapelte Verbandmaterialbehälter 10 dargestellt, die im geöffneten Zustand ineinander gelegt wurden. In der gepunkteten Ansicht ist jeweils der unterste Behälter 10 geschlossen weitergeführt sowie ein möglicherweise letzter aufzustapelnder Behälter 10, wobei dann jeweils in jede Halbschale 12 bzw. 14 noch ein geschlossener Verbandmaterialkasten 10 zum Abschluß des Stapels eingefügt werden kann.

Die Stapelbarkeit wird durch die gerundete Schalengestaltung verbessert.
Aufgrund der Stapelbarkeit des Behälters in seinem geöffneten Leerzustand lassen sich die erfindungsgemäßen Verbandmaterialbehältnisse 10 besonders platzsparend im Leerzustand transportieren. Es wird hierfür nur ein geringer Paletten- und Gitterkastenplatz benötigt.

Die Filmscharniere 18 sowie 26 können dabei so weit belastet und dadurch verschwenkt werden, daß im Bereich des Filscharnieres 18 sowohl das Behälteroberteil 12 als auch das Behälterunterteil 14 auf einer Unterlage aufliegen. Das Filmscharnier 26 wird so verschwenkt, daß die Lasche 20 parallel zur Unterlage 32 verläuft. Die Stapelung kann dabei derart erfolgen, daß jeweils auf ein Behälterunterteil 14 ein Behälteroberteil 12 gelegt wird und so fort.

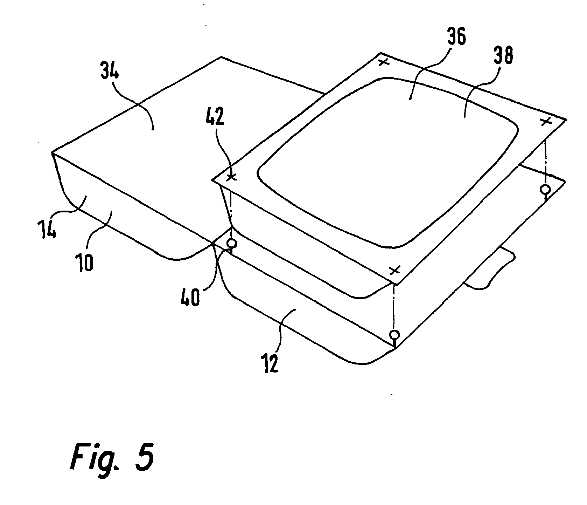

Die verbleibende Figur 5 zeigt in schematischer Darstellung einen Verbandmaterialbehälter 10, in den im Bereich des Behälterunterteils 14 bereits eine Inhaltsverpackung 36 für Verbandmaterial eingebracht wurde. Die Inhaltsverpackung 36, die in das Behälteroberteil 12 eingesetzt wird, ist in der noch nicht eingefügten Position dargestellt. Das Verbandsmaterial (nicht dargestellt) ist dabei in Inhaltsverpackungen 36 verpackt, um es zum einen besser handhabbar zu machen und zum anderen um es vor Verschmutzung zu schützen. Diese Inhaltsverpackungen 36 weisen an ihrer Oberseite 38 Beschriftungen auf, denen zu entnehmen ist, wozu die darin befindlichen Produkte dienen, so beispielsweise auf der einen Seite Produkte zur Fixierung und sonstiges Erste-Hilfe-Material und auf der anderen Seite im Behälterunterteil Produkte zur Wundversorgung. Diese Produkte können dann im Folgenden einzeln aufgelistet und eventuell auch bildlich erläutert sein. Weitere Erläuterungen wie beispielsweise Verfallsdatum und Verwendungshinweise können angebracht sein.

Die Inhaltsverpackungen 36 werden dann in die jeweiligen Halbschalen eingelegt, wobei in den Halbschalen Dorne 40 angeordnet sind, die Kreutzschlitzstanzungen 42 in den Inhaltsverpackungen 36 durchgreifen und mit diesen eine formschlüssige Halterung bilden. Die Inhaltsverpackungen 36 sind auf diese Weise in der jeweiligen Halbschale gesichert.

Ein einfaches Austauschen auch einzelner Inhaltsverpackungen 36 ist auf diese Weise möglich. So können die Inhaltsverpackungen nach Ablauf des Verfallsdatums, aber auch da Materialien hieraus verbraucht worden sind, ausgetauscht und der Verbandskasten so ergänzt werden.

Die beiden Inhaltsverpackungen 36 stehen im aufgeklappten Zustand beide gleichzeitig dem Nutzer zur Verfügung und es ist daher nicht notwendig, die Verbandmaterialien in mehreren Lagen übereinander im Verbandskasten anzuordnen. Die Verbandmaterialien können dabei über eine größere Oberfläche angeordnet und dargeboten sein, wodurch die Erstversorgung erleichtert wird.

## Patentansprüche

1. Kraftfahrzeug-Verbandmaterialbehälter mit Erste-Hilfe-Material befüllbar, umfassend einen Behälterunterteil (14) zur Aufnahme von Erste-Hilfe-Material mit einem Behälterboden (14e) und daran anschließenden Seitenwänden (14a-d) zur Bildung eines Behälters sowie einem Behälterdeckel (12e), der an dem Behälterunterteil (14) angelenkt ist und zwischen einer geschlossenen Position, in der er auf dem Behälterunterteil (14) aufliegt und dieses verschließt, und einer geöffneten Position, in der das Behälterunterteil (14) zugänglich ist, verschwenkbar ist, und wobei der Behälterdeckel (12e) mit dem Behälterunterteil (14) in der geschlossenen Position lösbar verriegelbar ist, der Behälterdeckel (12e) als Behälteroberteil (12) zur Aufnahme von Erste-Hilfe-Material ausgebildet ist und in dem Behälteroberteil (12) befindliches Erste-Hilfe-Material beim Öffnen des Verbandmaterialbehälters (10) mit dem Behälteroberteil (14) verschwenkbar ist, **dadurch gekennzeichnet, dass** das Behälteroberteil (12) und das Behälterunterteil (14) schalenförmig verrundet als zwei Halbschalen ausgebildet sind, so dass der Verbandmaterialbehälter (10) im unbefüllten Zustand ineinander stapelbar ist.

2. Kraftfahrzeug-Verbandmaterialbehälter nach Anspruch 1, **dadurch gekennzeichnet, daß** das in dem Behälteroberteil (12) und/oder Behälterunterteil (14) angeordnete Verbandmaterial in dem jeweiligen Behälterteil (12, 14) herausnehmbar festgelegt ist.

3. Kraftfahrzeug-Verbandmaterialbehälter nach Anspruch 1 oder 2, **dadurch gekennzeichnet, daß** das Verbandmaterial in Inhaltsverpackungen (36) angeordnet ist, die kraft-, form- oder materialschlüssig mit dem jeweiligen Behälterteil (12, 14) verbunden sind.

4. Kraftfahrzeug-Verbandmaterialbehälter nach Anspruch 3, **dadurch gekennzeichnet, daß** die Verbindung mittels Druckknöpfen, Haftkleber, Klettverbindungen oder Kreutzschlitz/Dornkombinationen (40, 42) erfolgt.

5. Kraftfahrzeug-Verbandmaterialbehälter nach einem oder mehreren der Ansprüche 1 bis 4, **dadurch gekennzeichnet, daß** das Behälteroberteil (12) bzw. das Behälterunterteil (14) einen Behälterdeckel (12e) bzw. einen Behälterboden (14e) sowie vier Seitenwände (12a-d; 14a-d) umfassen und daß die korrespondierenden Seitenwände (12a-d; 14a-d) des Behälterunterteils (14) und des Behälteroberteils (12) im Bereich ihrer freien Kanten im geschlossenen Zustand des Behälters (10) zusammenwirken.

6. Kraftfahrzeug-Verbandmaterialbehälter nach einem oder mehreren der Ansprüche 1 bis 5, **dadurch gekennzeichnet, daß** das Behälteroberteil (12) mit dem Behälterunterteil (14) mittels einer formschlüssigen Verbindung lösbar verriegelbar ist.

7. Kraftfahrzeug-Verbandmaterialbehälter nach Anspruch 6, **dadurch gekennzeichnet, daß** ein Behälterteil (12, 14) mindestens eine Lasche (20) aufweist, die eine Rastnase (22) besitzt, die mit einer korrespondierenden Kante (24) des anderen Behälterteils (12, 14) schnappend, rastend oder in sonstiger Weise hintergreifend zusammenwirkt.

8. Kraftfahrzeug-Verbandmaterialbehälter nach einem oder mehreren der Ansprüche 1 bis 7, **dadurch gekennzeichnet, daß** das Behälterunterteil (14) und das Behälteroberteil (12) in wesentlichen identisch geformt sind, insbesondere im wesentlichen die gleichen Abmaße besitzen.

9. Kraftfahrzeug-Verbandmaterialbehälter nach einem oder mehreren der Ansprüche 1 bis 8, **dadurch gekennzeichnet, daß** das Behälterunterteil (14) und das Behälteroberteil (12) einstückig miteinander verbunden sind.

10. Kraftfahrzeug-Verbandmaterialbehälter nach Anspruch 9, **dadurch gekennzeichnet, daß** die gelenkige Verbindung zwischen Behälteroberteil und Behälterunterteil ein Filmscharnier (18) ist.

11. Kraftfahrzeug-Verbandmaterialbehälter nach einem oder mehreren der Ansprüche 1 bis 10, **dadurch gekennzeichnet, daß** die Lasche (20) zum Verriegeln von Behälteroberteil (12) und Behälterunterteil (14) mit dem entsprechenden Behälterteil (14, 12) einstückig über ein Filmscharnier (26) verbunden ist.

12. Kraftfahrzeug-Verbandmaterialbehälter nach einem oder mehreren der Ansprüche 1 bis 11, **dadurch gekennzeichnet, daß** der Verbandmaterialbehälter (10) ein Spritzgußteil ist.

13. Kraftfahrzeug-Verbandmaterialbehälter nach einem oder mehreren der Ansprüche 1 bis 12, **dadurch gekennzeichnet, daß** der Verbandmaterialbehälter (10) aus einem Polypropylenmaterial besteht.

## Claims

1. A motor-vehicle container for dressing material, which may be filled with first-aid material, and which for receiving first-aid material has a container lower part (14), which has a container base (14e) and side walls (14a-d) attached thereto so as to form a container, and a container cover (12e), which is hinged on the container lower part (14) and pivots between a closed position, in which it contacts the container lower part (14) and closes it, and an opened position, in which the container lower part (14) is accessible, and whereby the container cover (12e) in a closed position can be detachably locked to the container lower part (14), the container cover (12e) is configured as a container upper part (12) for receiving first-aid material, and the first-aid material that is located in the container upper part (12) can be pivoted along with the container upper part (14) when the dressing material container (10) is opened, wherein the container upper part (12) and the container lower part (14) are rounded in a bowl-shape fashion and are configured as two half shells, so that in the empty state the dressing material containers (10) can be stacked one inside the other.

2. The motor-vehicle container for dressing material as recited in Claim 1, wherein dressing material that is arranged in the container upper part (12) and/or container lower part (14) is secured in the respective container part (12, 14) so as to be removable.

3. The motor-vehicle container for dressing material as recited in Claim 1 or 2, wherein the dressing material is arranged in packaging (36), which is connected to the respective container part (12, 14) in a force-, form-, or material-locking manner.

4. The motor-vehicle container for dressing material as recited in Claim 3, wherein the connection is accomplished by push buttons, pressure-sensitive adhesives, velcro connections, or cross-slot/tab combinations (40, 42).

5. The motor-vehicle container for dressing material as recited in one or more of Claims 1 to 4, wherein the container upper part (12) or the container lower part (14) includes a container cover (12e) or a container base (14e) respectively as well as four side walls (12a-d; 14a-d), and, in the closed state of the container (10), the respective side walls (12a-d;14a-d) of the container lower part (14) and of the container upper part (12) cooperate in the area of their free edges.

6. The motor-vehicle container for dressing material as recited in one or more of Claims 1 to 5, wherein the container upper part (12) can be detachably locked to the container lower part (14) in a form-locking connection.

7. The motor-vehicle container for dressing material as recited in Claim 6, wherein a container part (12, 14) has at least one flap (20), which has a latch (22), which cooperates with a corresponding edge (24) of the other container part (12, 14) in a snapping, locking, or other manner, from the rear side.

8. The motor-vehicle container for dressing material as recited in one or more of Claims 1 to 7, wherein the container lower part (14) and the container upper part (12) are shaped so as to be essentially identical, and in particular to have essentially identical dimensions.

9. The motor-vehicle container for dressing material as recited in one or more of Claims 1 to 8, wherein the container lower part (14) and the container upper part (12) are integrally joined to each other.

10. The motor-vehicle container for dressing material as recited in Claim 9, wherein the articulated connection between the container upper part and the container lower part is a film hinge (18).

11. The motor-vehicle container for dressing material as recited in one or more of Claims 1 to 10, wherein the flap (20) for locking the container upper part (12) and the container lower part (14) is integrally connected to the corresponding respective container part (14, 12) by a film hinge (26).

12. The motor-vehicle container for dressing material as recited in one or more of Claims 1 to 11, wherein the dressing material container (10) is an injection-molded part.

13. The motor-vehicle container for dressing material as recited in one or more of Claims 1 to 12, wherein the dressing material container (10) is made of a polypropylene material.

## Revendications

1. Contenant de matériel de pansement de véhicule pouvant être rempli avec des matières de premiers soins, comportant un élément inférieur de contenant (14) permettant de loger les matières de premiers soins avec un fond de contenant (14e) et des parois latérales (14a à d) adjacentes à celui-ci permettant de former un contenant ainsi qu'un couvercle de contenant (12e) qui est articulé sur l'élément inférieur de contenant (14) et peut être pivoté entre une position fermée dans laquelle il repose sur l'élément inférieur de contenant (14) et ferme celui-ci, et une position ouverte dans laquelle l'élément inférieur de contenant (14) est accessible, et moyennant quoi le couvercle de contenant (12e) peut être bloqué de manière amovible avec l'élément inférieur de contenant (14) dans la position fermée, le couvercle de contenant (12e) est configuré sous la forme d'un élément supérieur de contenant (12) permettant de loger les matières de premiers soins et les matières de premiers soins se trouvant dans l'élément supérieur de contenant (12) peuvent être pivotées avec l'élément supérieur de contenant (14) lors de l'ouverture du contenant de matières de pansement (10), **caractérisé en ce que** l'élément supérieur de contenant. (12) et l'élément inférieur de contenant (14) sont arrondis sous la forme d'une coque et sont configurés sous la forme de deux demi-coques, de telle sorte que le contenant de matières de pansement (10) peut être empilé l'un sur l'autre dans l'état non rempli.

2. Contenant de matières de pansement de véhicule selon la revendication 1, **caractérisé en ce que** la matière de pansement disposée dans l'élément supérieur de contenant (12) et/ou dans l'élément inférieur de contenant (14) est fixée de manière à pouvoir être retirée dans l'élément de contenant (12, 14) respectif.

3. Contenant de matières de pansement de véhicule selon l'une quelconque des revendications 1 ou 2, **caractérisé en ce que** la matière de pansement est disposée dans des emballages de contenu (36), qui sont reliés par conjugaison de force, de forme ou de matière avec l'élément de contenant respectif (12, 14).

4. Contenant de matières de pansement de véhicule selon la revendication 3, **caractérisé en ce que** l'assemblage est réalisé par boutons-pression, adhésif de contact, attaches autoagrippantes ou combinaisons de fente cruciforme/tourillon (40, 42).

5. Contenant de matières de pansement de véhicule selon une ou plusieurs des revendications 1 à 4, **caractérisé en ce que** l'élément supérieur de contenant (12) ou l'élément inférieur de contenant (14) comporte un couvercle de contenant (12e) ou un fond de contenant (14e) et quatre parois latérales (12a à d ; 14a à d) et **en ce que** les parois latérales correspondantes (12a à d ; 14a à d) de l'élément inférieur de contenant (14) et de l'élément supérieur de contenant (12) coopèrent dans la zone de leurs bords libres dans l'état fermé du contenant (10).

6. Contenant de matières de pansement de véhicule selon une ou plusieurs des revendications 1 à 5, **caractérisé en ce que** l'élément supérieur de contenant (12) peut être bloqué de manière amovible avec l'élément inférieur de contenant (14) à l'aide d'un assemblage par conjugaison de forme.

7. Contenant de matières de pansement de véhicule selon la revendication 6, **caractérisé en ce qu'**un élément de contenant (12, 14) présente au moins un collier (20) qui possède un cran (22) lequel coopère avec un bord correspondant (24) de l'autre élément de contenant (12, 14) par enclenchement, encliquetage, ou d'une autre manière en mordant par l'arrière.

8. Contenant de matières de pansement de véhicule selon une ou plusieurs des revendications 1 à 7, **caractérisé en ce que** l'élément inférieur de contenant (14) et l'élément supérieur de contenant (12) sont formés de manière sensiblement identique, en présentant sensiblement les mêmes dimensions.

9. Contenant de matières de pansement de véhicule selon une ou plusieurs des revendications 1 à 8, **caractérisé en ce que** l'élément inférieur de contenant (14) et l'élément supérieur de contenant (12) sont assemblés d'un seul tenant l'un à l'autre.

10. Contenant de matières de pansement de véhicule selon la revendication 9, **caractérisé en ce que** l'assemblage articulé entre l'élément supérieur de contenant et l'élément inférieur de contenant est un film charnière (18).

11. Contenant de matières de pansement de véhicule selon une ou plusieurs des revendications 1 à 10, **caractérisé en ce que** le collier (20) est assemblé d'un seul tenant à l'élément de contenant correspondant (14, 12) par l'intermédiaire d'un film charnière (26) afin de bloquer l'élément supérieur de contenant (12) et l'élément inférieur de contenant (14).

12. Contenant de matières de pansement de véhicule selon une ou plusieurs des revendications 1 à 11, **caractérisé en ce que** le contenant de matières de pansement (10) est un élément moulé par injection.

13. Contenant de matières de pansement de véhicule selon une ou plusieurs des revendications 1 à 12, **caractérisé en ce que** le contenant de matières de pansement (10) est constitué d'une matière en polypropylène.
